(19) Europäisches Patentamt
European Patent Office
Office européen des brevets

(11) Publication number: 0 401 946 A2

# (12) EUROPEAN PATENT APPLICATION

(21) Application number: 90250143.6

(51) Int. Cl.5: C07C 69/734, A01N 37/38

(22) Date of filing: 01.06.90

(30) Priority: 05.06.89 DE 3918635

(43) Date of publication of application: 12.12.90 Bulletin 90/50

(84) Designated Contracting States: AT BE CH DE DK ES FR GB GR IT LI LU NL SE

(71) Applicant: **SCHERING AKTIENGESELLSCHAFT**
**Müllerstrasse 170/178**
**D-1000 Berlin 65(DE)**

(72) Inventor: **Bühmann, Ulrich, Dr.**
**Am Volkspark 83**
**D-1000 Berlin 31(DE)**
Inventor: **Joppien, Hartmut, Dr.**
**Juttastrasse 18**
**D-1000 Berlin 37(DE)**
Inventor: **Keyserlingk, Harald V., Dr.**
**Markgrafenstrasse 38**
**D-1000 Berlin 28(DE)**

## (54) Allyl esters of substituted phenylacetic acid and their use as pesticides.

(57) There are described new allyl esters of substituted phenylacetic acid of general formula I

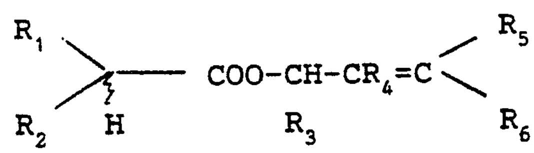

(I)

in which
$R_{1-6}$ have the meanings given in the description as well as processes for their preparation. The compounds can be used as pesticides especially against insects and acarids.

EP 0 401 946 A2

**ALLYL ESTERS OF SUBSTITUTED PHENYLACETIC ACID AND THEIR USE AS PESTICIDES**

This invention relates to new allyl esters of substituted phenylacetic acid, processes for their preparation and their use as pesticides with insecticidal and acaricidal activity.

Arylacetic acid esters with insecticidal and acaricidal activity are known (DE OS 26 47 366)

The object of the present invention is to provide arylacetic acid esters that have a better activity with greater selectivity.

It has now been found that substituted allyl esters of phenylacetic acid of general formula I

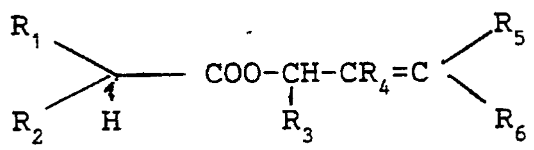

(I)

in which

$R_1$ is phenyl, optionally substituted by one or more of the same or different groups selected from halogen, $C_{1-4}$-alkyl, $C_{3-6}$-cycloalkyl, $C_{2-6}$-alkenyl, $C_{2-6}$-alkynyl, $C_{1-4}$-alkoxy, $C_{3-6}$-alkenyloxy, $C_{2-6}$-alkynyloxy, $C_{3-6}$-cycloalkoxy, $C_{3-6}$-cycloalkylmethoxy, $C_{1-4}$-alkylthio, $C_{1-4}$-alkylsulfinyl, $C_{1-4}$-alkylsulfonyl, phenoxy, $C_{1-4}$-alkylsulfonyloxy, (each of which is optionally substituted by halogen), halogen, cyano and nitro;

$R_2$ is tert-butyl or 1-methylcyclopropyl;

$R_3$ hydrogen, $C_{1-4}$-alkyl, optionally substituted by halogen, $C_{2-4}$-alkenyl, optionally substituted by halogen, halogen or cyano; and

$R_4$, $R_5$ and $R_6$ are the same or different and are hydrogen or halogen or the group $C_{1-4}$-alkyl, $C_{3-6}$-cycloalkyl, $C_{2-6}$-alkenyl, $C_{2-6}$-alkynyl or $C_{1-4}$-alkoxy, (each of which is optionally substituted by halogen), have an improved activity compared with known arylacetic acid esters.

By the term halogen is to be understood fluorine, chlorine, bromine and iodine. The designation "halo" in compounds with the groups alkyl, cycloalkyl, alkoxy, alkylthio, alkenyl, alkynyl, alkenyloxy, alkynyloxy, cycloalkoxy, cycloalkylmethoxy, alkylsulfonyl, alkylsulfinyl, phenyl or phenoxy means that one or more hydrogen atoms are replaced by halogen.

The invention incudes the individual isomers of the compounds of formula I as well as their mixtures.

The compounds of the invention can be prepared according to known processes. One process comprises the treatment of a compound of formula II

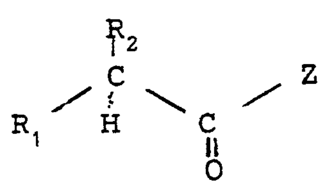

(II)

in which $R_1$ and $R_2$ have the meanings given above and Z is hydroxy or hydroxy, with an alcohol of general formula III

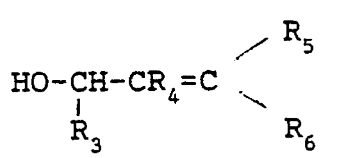

(III)

in which $R_3$, $R_4$, $R_5$ and $R_6$ have the meanings given in formula I above.

In the case when compounds of general formula II in which Z = halogen, are reacted, this is an acylation of an alcohol of formula III, in which Y is OH, with a carboxylic acid halide (of. e.g. "Reaktionen und Synthesen im organish chemischen Praktikum, L.F. Tietze - Th. Eicher, Thieme Verlag Stuttgart, 1981, page 115).

The reaction is suitably carried out in the presence of an acid acceptor (of. "Houben-Weyl, Methoden

der organischen Chemie", Band VIII, p. 541 ff, Georg Thieme Verlag, Stuttgart 1952).

Conventional basic materials are suitable as acid acceptors, such as for example aliphatic, aromatic and heterocyclic amines, e.g. triethylamine, dimethylamine and pyridine. The reaction can be carried out with or without a solvent. In addition to the acid acceptors, suitable solvents or their mixtures include optionally chlorinated aliphatic and aromatic hydrocarbons, such as petroleum ether, benzene, toluene, xylene, dichloromethane, chloroform, carbon tetrachloride, 1,2-dichloroethane and chlorobenzene; ethers, such as diethyl and di-n-butyl ether, methyl tert-butyl ether, tetrahydrofuran and dioxane; ketones, such as acetone, methyl ethyl ketone and methyl isopropyl ketone, and nitriles, such as acetonitrile and propionitrile.

Generally the reaction materials are used in stoichiometric amounts. An excess of one or more can be used in some cases but generally without advantage.

The reaction is generally carried out at a temperature above 0° with suffifient speed. Since heat is usually generated, it is often advantageous to provide cooling.

In the case when compounds of general formula II in which Z = hydroxy, are reacted with a compound of general formula III, in which Z = hydroxy, this is an esterification of a carboxylic acid (of. Houben-Weyl, Methoden der organischen Chemie, Band VIII, p. 516 ff, Georg Thieme Verlag," Stuttgart 1952), which can be speeded up in a known way, optionally by addition of catalysts, such as sulphuric acid, hydrogen halides or sulphonic acids or acid ion exchange resins, and the esterification equilibrium can be pushed into the desired direction by removal, from the reaction mixture, of water or the ester of general formula I, such as for example by azeotropic distillation.

The compounds of the invention of general formula I can also be synthesised by practically all conventional methods for preparing esters, eg by using carboxylic acid anhydrides, that can be obtained from the carboxylic acids of general formula II, or by reaction of salts of these carboxylic acids with compounds of general formula IV

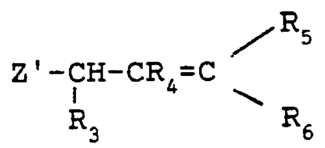

(IV)

in which $R_3$, $R_4$, $R_5$ and $R_6$ have the meanings given above and Z′ is halogen.

The phenylacetic acids used as starting materials of general formula II, in which Z = OH are generally known. They can be prepared according to known processes (see for example J. Org. Chem. 32 (9) 1967, 2799 and 2801 and Chem. Ber. 116 (1983), 3708-3724).

The optionally used phenylacetyl halides of general formula II, in which Z is halogen, can be prepared from these free acids by conventional methods.

The alcohols of general formula III are generally commercial products or can be prepared according to generally known methods.

One process, especially for the preparation of compounds of general formula I with a Z-alkenol component, comprises the partial hydrogenation of alkynyl esters using palladium with a trace of a heavy metal salt, eg the Lindlar catalyst, by which the CC-triple bond alone is reduced to the CC-double bond.

Suitable alkynyl esters used as starting materials are described for example in EP 261 072.

The compounds of the invention are, as a rule, colourless oils that are highly soluble in practically all organic solvents but are almost insoluble in water.

The compounds of the invention have insecticidal and acaricidal activity and as a result can be used for combating a wide range of insects and acarids, including animal ectoparasites. Examples include Lepidoptera, such as Plutella xylostella, Spodoptera littoralis, Heliothis armigera and Pieris brassicae; Diptera, such as Musca domestica, Ceratitis capitata, Erioischia brassicae, Lucilia sericata and Aedes aegypti; Homoptera, including aphids such as Megoura viciae and Nilaparvata lugens; Coleoptera, such as Phaedon cochleariae, Anthonomus grandis and corn rootworms (Diabrotica spp. eg. Diabrotica undecimpunctata); Orthoptera, such as Blattella germanica; ticks, such as Boophilus microplus and lice, such as Damalinia bovis and Linognathus vituli, as well as spider mites such as Tetranychus urticae and Panonychus ulmi.

The compounds of the invention are suitable in a surprising manner for combating insects, especially combating pest insects and represent a valuable improvement in the state of the art.

The compounds according to the invention can be used at a concentration of 0.0005 to 5%, preferably from 0.001 to 1%, calculated as gram active material per 100 ml of the composition.

The compounds of the invention can be used either alone or in mixture with each other or another insecticide. Optionally other plant protection or pesticidal compositions, such as for example insecticides,

acaricides or fungicides can be added depending on the desired result.

An improvement in the intensity and speed of action can be obtained, for example, by addition of suitable adjuvants, such as organic solvents, wetting agents and oils. Such additives may allow a decrease in the dose.

Suitable mixture partners may also include phospholipids, e.g. such as from the group phosphatidylcholine, hydrated phosphatidylcholine, phosphatidylethanolamine, N-acyl-phosphatidylethanolamine, phosphatidylinositol, phosphatidylserine, lysolecithin or phosphatidylglycerol.

The designated active ingredients or their mixtures can suitably be used, for example, as powders, dusts, granules, solutions, emulsions or suspensions, with the addition of liquid and/or solid carriers and/or diluents and, optionally, binding, wetting, emulsifying and/or dispersing adjuvants.

Suitable liquid carriers are, for example aliphatic and aromatic hydrocarbons such as benzene, toluene, xylene, cyclohexanone, isophorone, dimethyl sulphoxide, dimethylformamide, other mineral-oil fractions and plant oils.

Suitable solid carriers include mineral earths, e.g. tonsil, silica gel, talcum, kaolin, attapulgite, limestone, silicic acid and plant products, e.g. flours.

As surface-active agents there can be used for example calcium lignosulphonate, polyoxyethylenealkylphenyl ether, naphthalenesulphonic acids and their salts, phenolsulphonic acids and their salts, formaldehyde condensates, fatty alcohol sulphates, as well as substituted benzenesulphonic acids and their salts.

Formulations can be prepared, for example, from the following ingredients.

A WETTABLE POWDER

20 percent by weight active ingredient
35 percent by weight bentonite
8 percent by weight calcium lignosulphonate
2 percent by weight of the sodium salt of N-methyl-N-oleyltaurine
35 percent by weight silicic acid

B PASTE

45 percent by weight active ingredient
5 percent by weight sodium aluminium silicate
15 percent by weight cetylpolyglycol ether with 8 moles ethylene oxide
2 percent by weight spindle oil
10 percent by weight polyethylene glycol
23 parts water

C EMULSIFIABLE CONCENTRATE

20 percent by weight active ingredient
75 percent by weight isophorone
5 percent by weight of a mixture of nonylphenylpolyoxyethylene and calcium dodecylbenzenesulphonate

The following examples illustrate the preparation of compounds according to the invention.

## Example 1

(E)-2-Butenyl 2-(4-ethoxy-3-fluorophenyl)-3,3-dimethylbutyrate

100 mg 4-Dimethylaminopyridine and 4.1 g (15 mmol) 2-(4-ethoxy-3-fluorophenyl)-3,3-dimethylbutyryl chloride were added to a solution of 1.08 g (15 mmol) trans-crotyl alcohol in 15 ml pyridine at 0 °C (ice cooling). The mixture was stirred at room temperature for approx 18 hours and then poured into ice-water. After acidification with dilute hydrochloric acid, the mixture was extracted several times with dichloromethane. The organic phase was washed with water, dried over magnesium sulphate and concentrated under reduced pressure. The resulting oil was purified by thin layer chromatography. There was obtained an almost colourless oil.

Yield: 3.6g = 78.9% of theory

$n^{20}_D$ : 1.4961

## Example 2

2-Bromo-2-propenyl 2-(4-ethoxy-3-fluorophenyl)-3,3-dimethylbutyrate

5.1 g (0.02 mmol) 2-(4-Ethoxy-3-fluorophenyl)-3,3-dimethylbutyric acid, dissolved in 80 ml dry dimethylformamide, was added, under ice cooling, dropwise, to a suspension of 0.66 g (0.022 mmol) 80% sodium hydride in 20 ml dry dimethylformamide at 0 °C. After hydrogen evolution had slowed down, the reaction mixture was treated with 4.4 g (0.02 mmol) 2,3-dibromo-1-propene. The mixture was then warmed for 3 hours at 60 °C. It was then added to ice-water and extracted several times with hexane. After drying the hexane phase over magnesium sulphate, it was filtered and concentrated under reduced pressure. The remaining oil was chromatographed on silica gel.
Yield: 4.99 g = 66.8% of theory of an almost colourless oil.
$n^{20}_D$ : 1.5169

Example 26

(Z)-2-Butenyl 2-(4-ethoxy-3 fluorophenyl)-3,3-dimethylbutyrate

2.5 g (8.16 mmol) 2-Butinyl (Z)-2-(4-ethoxy-3-fluorophenyl)-3,3-dimethylbutyrate was dissolved in 25 ml ether and put into an shaking flask. 250 mg Lindlar catalyst (Aldrich Europe, D-4054 Nettetal 2, Type 20, 573-7) was added and the compound hydrogenated at a slight excess pressure until about 180 ml hydrogen had been taken up. After filtering from the catalyst, the mixture was concentrated and chromatographed on silica gel.

There was obtained 2.32 g = 92.2% of theory of an almost colourless oil.
$n^{20}_D$ : 1.4988

In a similar way the following compounds were prepared.

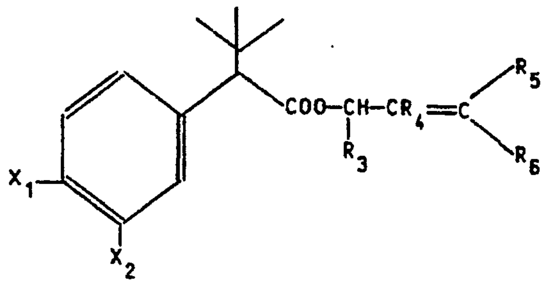

| Example No | $X_1$ | $X_2$ | $R_3$ | $R_4$ | $R_5$ | $R_6$ | $n_D^{20}$ |
|---|---|---|---|---|---|---|---|
| 3 | $OC_2H_5$ | H | H | H | H | H | 1,502 |
| 4 | Cl | H | H | H | H | H | 1,31 |
| 5 | Cl | H | H | Br | H | Br | 1,5587 *) |
| 6 | Cl | H | $-CH=CH_2$ | H | H | H | 1,5112 |
| 7 | Cl | H | H | Br | H | H | 1,5345 |
| 8 | $OC_2H_5$ | F | H | H | H | H | 1,4919 |
| 9 | $OC_2H_5$ | F | $-CH=CH_2$ | H | H | H | 1,4974 |
| 10 | $OC_2H_5$ | F | H | Br | H | Br | 1,5387 *) |
| 11 | $OC_2H_5$ | F | H | $CH_3$ | H | H | 1,4947 |
| 12 | $OC_2H_5$ | F | H | H | $CH_3$ | $CH_3$ | 1,4977 |
| 13 | $OC_2H_5$ | H | H | Br | H | H | 1,5237 |
| 14 | Cl | H | H | $CH_3$ | H | H | 1,5093 |
| 15 | Cl | H | H | H | H | $CH_3$ | 1,5094 ***) |
| 16 | Cl | H | H | H | $CH_3$ | $CH_3$ | 1,5120 |
| 17 | $OC_2H_5$ | F | H | H | $CH_3$ | $-C\equiv CH$ | 1,5099 **) |
| 18 | $OC_2H_5$ | H | H | H | $CH_3$ | $-C\equiv CH$ | 1,5188 **) |
| 19 | Cl | H | H | H | $CH_3$ | $-C\equiv CH$ | 1,5265 **) |
| 20 | $OC_2H_5$ | F | H | H | H | $-C\equiv CH$ | 1,5127 *) |
| 21 | $OC_2H_5$ | H | H | H | H | $-C\equiv CH$ | 1,5226 *) |
| 22 | Cl | H | H | H | H | $-C\equiv CH$ | 1,5306 *) |
| 23 | Cl | H | H | H | $CH_3$ | $-(CH_2)_2-CH=C(CH_3)-CH_3$ | 1,5135 |

| Example No | $X_1$ | $X_2$ | $R_3$ | $R_4$ | $R_5$ | $R_6$ | $n_D^{20}$ |
|---|---|---|---|---|---|---|---|
| 24 | $OC_2H_5$ | H | H | H | H | $CH_3$ | 1,5027 ***) |
| 25 | $OC_2H_5$ | H | H | H | H | $CH_3$ | 1,5053 **) |
| 27 | $CF_3$ | H | H | H | H | $CH_3$ | 1,4640 ***) |
| 28 | $-OCF_3$ | H | H | H | $CH_3$ | H | 1,4619 **) |
| 29 | $-OCF_3$ | H | H | H | $CH_3$ | H | 1,4608 ***) |
| 30 | $-OCF_3$ | H | H | H | H | H | 1,4556 |
| 31 | $-CF_3$ | H | H | H | $CH_3$ | H | 1,4690 **) |
| 32 | Cl | H | -C≡N | H | $CH_3$ | H | 1,5145 A) ***) |
| 33 | Cl | H | -C≡N | H | $CH_3$ | H | 1,5141 B) ***) |
| 34 | $OC_2H_5$ | F | -C≡N | H | H | H | 1,4889 C) |
| 35 | $OC_2H_5$ | F | -C≡N | H | $CH_3$ | H | 1,4993 C) ***) |

*) E/Z-mixture
**) Z-isomer
***) E-isomer
A) isomerism: Diastereomer A
B) isomerism: Diastereomer B
C) isomerism: Diastereomer mixture 1:1

The following test Examples demonstrate the biological activity of the compounds of the invention.

Use Example A

Activity in the prophylactic treatment of feed against the against black bean aphids (Aphis fabae Scop.)

From the primary leaf of field beans (Phaseolus vulgaris nanus Aschers.), 24 mm diameter discs were cut. Some of these were treated with a 0.1% aqueous preparations of compounds of the invention and these along side untreated discs were placed on filter papers with the underside of the leaves turned upwards. After drying the test pieces, they were each infested with wingless stages of Aphis fabae (approx 100 per leaf piece). The experiment was replicated 3 times. The leaves were kept on wet filter papers for 2 days at 25°C and 16 hours light per day. The percentage mortality was then estimated and the activity calculated using Abbott's method in comparison with the untreated controls.

The compounds of Examples 1-3, 8-13, 17, 19-22, 24, 26 and 32-35 showed an activity of 80% or more.

Use Example B

Activity in prophylactic treatment of leaves against brown rice-hoppers (Nilaparvata lugens Stal)

Rice seedlings (Oryzae sativa L.) in the two leaf stage (about 10 per polystyrene pot of size 6.5 x 6.5 cm) were either untreated or dipped until dripping wet, with an aqueous preparation containing 0.1% of active material. After drying the sprayed leaves, a transparent cylinder was placed over each pot and through an opening, about 30 brown rice-hoppers (Nilaparvata lugens) in the 4-5 stage, anaesthetised with carbon dioxide, were introduced into each pot. After closing the opening with a fine mesh screen, the pots were kept for 2 days at 28 °C and 16 hours/day of light in the glasshouse, the amount of dead hoppers was determined. The percentage mortality was then estimated and the activity calculated using Abbott's method in comparison with the untreated controls.

The compounds of Examples 1-3, 15-22 and 24-35 showed an activity of 80% or more.

Use Example C

Activity in the prophylactic treatment of feed against the two spotted mite (Tetranychus urticae Koch)

From the developed primary leaf of field beans (Phaseolus vulgaris nanus Aschers.) 14 mm diameter discs were cut. Some of these were treated with a 0.1% aqueous preparations of compounds of the invention and these along side untreated discs were placed on filter papers with the underside of the leaves turned upwards. After drying the test pieces, they were each infested with six adult female Tetranychus urticae and maintained for 3 days at 25 °C and 16 hours light per day. The experiment was replicated 4 times. Dead and alive females were then counted and removed. Similarly the number of eggs laid were counted. After a further 7 days, the number of living larvae were counted. The activity was calculated using Abbott's method in comparison with the untreated controls.

The compounds of Examples 2, 7, 9, 10, 14, 15, 18, 17-20, 22 and 32-35 showed 80-100% activity.

Use Example D

Activity against eggs/larvae of the corn rootworm (Diabrotica undecimpunctata)

The compounds of the invention were made up as aqueous emulsions at a concentration of 0.1%. Into the soil in polystyrene petri dishes, containing maize seedlings (1 seedling/dish) and ca. 50 eggs of the corn rootworm (Diabrotica undecimpunctata) were pipetted 0.2 ml of these preparations. The closed dishes were left at 25 °C under extended daylight conditions for 7 days. The criterion for judging the activity was the death of eggs or newly hatched larvae at the end of the test.

The compounds of Examples 1-22 and 24-35 showed 80-100% activity.

Use Example E

Soil insecticide activity against eggs/larvae of the corn rootworm (Diabrotica undecimpunctata)

Formulations of compounds of the invention were made up as aqueous emulsions containing 0.0025% of active ingredient. 5 ml of this preparation was pipetted into clear plastic beakers (300 ml) each of contained 50 $cm^3$ earth and ca. 50 Diabrotica eggs and 2 pre-soaked grains of corn. The pots were left in the glasshouse under extended daylight conditions at 25 °C for 10 days. The criterion for judging the activity after ten days was the larvicidal activity and the root growth of the emerging maize plants in the treated beakers in comparison with untreated controls.

The compounds of Examples 1, 8, 24, 26, 27, and 32-35 showed 90-100% larvicidal activity and good root growth.

Use Example F

Activity against larvae (L1) of the cotton bollworm (Heliothis viriscens)

Compounds of the invention were made up as aqueous preparations at a concentration of 0.1%. Into these, feed material was dipped for 2 seconds. After drying the feed material was put into into polystyrene petri dishes. After an hour, 10 L1 of the cotton bollworm (Heliothis viriscens) were counted into the dishes. The closed dishes were left for up to 7 days at 25 °C under extended daylight conditions. The % mortality of the larvae after two days indicated the level of activity.

The compounds of Examples 1-6, 8, 10, 12, 13, 15, 17-22, 24, 27, 29-31, 34 and 35 showed 80 - 100% activity.

Use Example G

Insecticidal activity against sheep blowfly (Lucilia sericata)

1 ml aliquots of an acetone solution containing test compound at various concentrations were applied to cotton wool dental rolls 1 cm x 2 cm, contained in glass vials (2 cm diameter x 5 cm long). After drying, the treated materials were then impregnated with 1ml of nutrient solution, infested with first instar larvae of sheep blowfly (Lucilia sericata), closed by a cotton wool plug and held at 25 °C for 24 hours.

For the controls the mortality was <5% whereas the compounds of Examples 1-6, 8, 9, 11-15, 17-22, 24-27, and 32-35 had an $LC_{50}$ of 300 ppm or less.

Use Example H

Insecticidal activity against house flies (Musca domestica)

Aliquots of acetone solutions of test compounds at various concentrations were applied to 9 cm diameter filter papers placed in the bottom of 9 cm diameter petri dishes closed by glass lids. After evaporation of solvent, the treated surfaces, together with control treated with acetone alone, were then infested with adult houseflies, (Musca domestica) and held at 22 °C for 24 hours. The percentage mortality of the insects was then recorded.

Less than 5% mortality resulted in the control treatments whereas the compounds of Examples 1-6, 8-9, 11-13, 15-22, 24-27, and 32-35 an $LC_{50}$ of 1000 $mg/m^2$ or less.

Use Example I

Activity against tick larvae (Boophilus microplus)

Filter papers (9 cm in diameter) were impregnated with 1 ml aliquots of acetone solutions of test compound at various concentrations. The papers were allowed to dry and then folded into envelopes in which cattle tick larvae, (Boophilus microplus) were enclosed and held at 25 °C and 80% R.H. for 48 hours. The percentage mortality of tick larvae was then recorded and compared with controls.

The controls gave a mortality of less than 5% whereas compounds of Examples 1, 4, 6, 8, 9, 19, 22, 24-27 and 32-35 caused 50% mortality at a concentration of 300 ppm or less.

## Claims

1. Allyl esters of substituted phenylacetic acid of general formula I

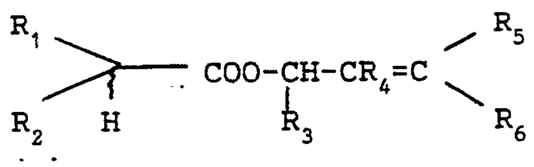

(I)

in which

$R_1$ is phenyl, optionally substituted by one or more of the same or different groups selected from halogen, $C_{1-4}$-alkyl, $C_{3-6}$-cycloalkyl, $C_{2-6}$-alkenyl, $C_{2-6}$-alkynyl, $C_{1-4}$-alkoxy, $C_{2-6}$-alkenyloxy, $C_{2-6}$-alkynyloxy, $C_{3-6}$-cycloalkoxy, $C_{3-6}$-cycloalkylmethoxy, $C_{1-4}$-alkylthio, $C_{1-4}$-alkylsulfinyl, $C_{1-4}$-alkylsulfonyl, phenoxy, $C_{1-4}$-alkylsulfonyloxy, (each of which is optionally substituted by halogen), halogen, cyano and nitro;

$R_2$ is tert-butyl or 1-methylcyclopropyl;

$R_3$ hydrogen, $C_{1-4}$-alkyl, optionally substituted by halogen, $C_{2-4}$-alkenyl, optionally substituted by halogen, halogen or cyano; and

$R_4$, $R_5$ and $R_6$ are the same or different and are hydrogen or halogen or the group $C_{1-4}$-alkyl, $C_{3-6}$-cycloalkyl, $C_{2-6}$-alkenyl, $C_{2-6}$-alkynyl or $C_{1-4}$-alkoxy, (each of which is optionally substituted by halogen),

2. An insecticidal and acaricidal composition which comprises a compound as claimed in claim 1 in admixture with an agriculturally acceptable diluent or carrier.

3. Use of a compound according to claim 1, for combating insects or acarids.

4. A method of combating insects and acarids which comprises applying to the insect or acarid or their locus, an effective amount of a compound claimed in claim 1.